# EUROPEAN PATENT SPECIFICATION

(11) **EP 3 930 452 B1**
(45) Date of publication and mention of the grant of the patent: **20.12.2023**
(21) Application number: 20762775.3
(22) Date of filing: 21.02.2020
(51) Int. Cl.: A01K 67/033

(54) **EGG HATCHING AND LARVAE SEPARATION DEVICE AND METHOD**
VORRICHTUNG UND VERFAHREN ZUR TRENNUNG VON BRUT UND LARVEN
DISPOSITIF ET PROCÉDÉ D'ÉCLOSION D'OEUFS ET DE SÉPARATION DE LARVES

(30) Priority: 28.02.2019 US 201916288287
(43) Date of publication of application: 05.01.2022
(73) Proprietor: Verily Life Sciences LLC, South San Francisco, CA 94080 (US)
(72) Inventor: METLITZ, Matthew, San Francisco, California 94080 (US); MASSARO, Peter, San Francisco, California 94080 (US); WHITE, Bradley, San Francisco, California 94080 (US)
(74) Representative: Mewburn Ellis LLP
(86) International application number: PCT/US2020/019235
(87) International publication number: WO 2020/176346

(56) References cited:
- WO-A1-2018/169398
- WO-A1-2018/169398
- WO-A1-2018/191276
- WO-A1-2018/191276
- CN-A- 107 996 520
- US-A- 2 804 045
- US-A- 2 804 045
- US-A- 3 223 237
- US-A- 3 682 138
- US-A- 3 682 138
- US-A1- 2004 194 717
- US-A1- 2004 194 717
- US-A1- 2013 273 599
- US-A1- 2013 273 599
- US-A1- 2014 020 630
- US-A1- 2015 305 312
- US-A1- 2015 305 312
- US-B1- 6 561 134
- US-B1- 6 561 134

## Description

### FIELD

The present disclosure relates generally to the mass-rearing of insects. More specifically, but not by way of limitation, this disclosure relates to devices and methods for separating insect larvae from egg hatching debris.

### BACKGROUND

The mass-rearing of insect larvae can be very time and labor intensive. To produce insect larvae, insect eggs are flooded in a solution (e.g., water plus yeast) and left for a number of hours in a container (e.g., a jar) at an appropriate temperature to allow for the desired number of larvae to hatch. A technician would then perform the difficult task of separating the hatched larvae from the egg hatching debris (e.g., the egg casings and the non-egg debris, such as mosquito body parts) in order to conduct a smooth and accurate larvae counting/allocation operation. Separating the hatched larvae from the egg hatching debris would typically be done by transferring the hatched larvae and the debris from the container to a tray for a technician to hand pluck the larvae from the egg hatching debris. This method often involved significant amounts of human labor and time, such as manually moving the hatched larvae and solution from the container to the tray, hand separating the hatched larvae from the egg casings and the non-egg debris, etc.

US 2 804 045 A describes a separating apparatus for hatching brine shrimp, comprising a rectangular tray having a flat bottom and vertical side walls. The tray is divided into three sections by diagonal partitions. An outlet pipe extends from one side of the tray.

CN1 07996520 A discloses a further device for hatching brine shrimps.

US3223237 A discloses a device for separating and classifying small water-borne insects such as mosquito.

### SUMMARY

Various examples are described for devices and methods for egg hatching and larvae separation. Accordingly, there is provided a device and a method according to the independent claims. One example device includes a fluid-tight container, wherein the fluid-tight container comprises a base having a first portion and a second portion; at least one wall enclosing the base and coupled to the base to form a fluid-tight seal; wherein the first portion of the base comprises a first color and defines a recess; wherein the recess is arranged for depositing of and keeping in place insect eggs for hatching in the first recess; and wherein the second portion of the base comprises a second color darker than the first color; wherein the color of the second portion is adapted to attract hatched insect larvae so that the hatched larvae move to the second portion and separate themselves from the egg debris located in the recess and in the first portion.

One example method includes providing a fluid-tight container and a liquid disposed in the fluid-tight container, wherein the fluid-tight container comprises a base having a first portion and a second portion; at least one wall enclosing the base and coupled to the base to form a fluid-tight seal; wherein the first portion of the base comprises a first color and defines a recess; and wherein the second portion of the base comprises a second color darker than the first color; dispensing a plurality of insect eggs into the recess of the fluid-tight container; and removing at least one larvae from the second portion of the fluid-tight container, the at least one larvae having hatched from an insect egg of the plurality of insect eggs.

These illustrative examples are mentioned not to limit or define the scope of this disclosure, but rather to provide examples to aid understanding thereof. Illustrative examples are discussed in the Detailed Description, which provides further description. Advantages offered by various examples may be further understood by examining this specification.

### BRIEF DESCRIPTION OF THE DRAWINGS

The accompanying drawings, which are incorporated into and constitute a part of this specification, illustrate one or more certain examples and, together with the description of the example, serve to explain the principles and implementations of the certain examples.
Figures 1-8 and 11 show example devices for egg hatching and larvae separation according to this disclosure;
Figure 9 shows a flowchart for an example method for egg hatching and larvae separation according to this disclosure; and
Figure 10 shows an example computing device for egg hatching and larvae separation according to this disclosure.

### DETAILED DESCRIPTION

Examples are described herein in the context of devices and methods for egg hatching and larvae separation. Those of ordinary skill in the art will realize that the following description is illustrative only and is not intended to be in any way limiting. Reference will now be made in detail to implementations of examples as illustrated in the accompanying drawings. The same reference indicators will be used throughout the drawings and the following description to refer to the same or like items.

In the interest of clarity, not all of the routine features of the examples described herein are shown and described. It will, of course, be appreciated that in the development of any such actual implementation, numerous implementation-specific decisions must be made in order to achieve the developer's specific goals, such as compliance with application- and business-related constraints, and that these specific goals will vary from one implementation to another and from one developer to another.

When mass rearing insects, it may be desirable to separate insect larvae (or simply "larvae") from egg debris without the need for manual separation by a user. Examples according to this disclosure can provide for the separation of insect larvae from insect egg hatching debris by the insect larvae themselves using the instinct of the larvae to flee from stimuli.

In an illustrative example, mosquito eggs are placed into a fluid-tight container that contains a liquid. This example fluid-tight container has a base that is divided into two adjacent sections: the first section is colored white and includes a recess where the mosquito eggs may be initially deposited, and a second section that is colored black. The base also has a slope from the recess up to the second portion. Thus, as the mosquito larvae hatch, they will move out of the recess, up the inclined slope, and into the second portion due to the larvae's inclination to hide in the black section because of the section's darker color.

In this example, the fluid-tight container may include various other features that may help encourage the larvae to leave the recess, thereby separating themselves from the egg hatching debris. The fluid-tight container has a light source that shines light into the first section of the container. Because mosquito larvae are startled by the light stimulus, they will move away from the light source and out of the first section.

In addition, the fluid-tight container has a vibration generator coupled to the first portion to output a mechanical vibration that disturbs the larvae and encourages them to move away from the vibration. The vibration may also encourage the hatched eggs and debris to settle, which may leave more room for the unhatched eggs. Additionally, a heat source, such as a heating pad, may be positioned under the first section along with a temperature measuring device, such as a temperature probe, in order to measure and maintain a temperature of the liquid in the container. Heating the first section may encourage the insect larvae eggs to hatch faster and more synchronously. A temperature gradient in the system due to heating the first section may cause the larvae to move toward the second section of the container. A computing device may be in communication with the light source, vibration generator, heat source, and temperature probe to control those devices.

Each of these features (e.g., the color of the first section versus the color of the second section, the light source, the vibration generator, and the heat source) would help to separate the larvae if used on its own. However, such a configuration as is described above incorporating each of the features into a single device encourages the larvae to hatch from the mosquito eggs, to leave the first section, and to move into the second section. As a result, the larvae separate themselves from the eggs and the egg hatching debris so a user does not have to manually separate each individual larvae from the debris. After the larvae have separated themselves from the debris, the larvae may be removed quickly and efficiently from the fluid-tight container and moved en masse into another rearing container.

This illustrative example is given to introduce the reader to the general subject matter discussed herein and the disclosure is not limited to this example. The following sections describe various additional non-limiting examples and examples of systems and methods for egg hatching and larvae separation.

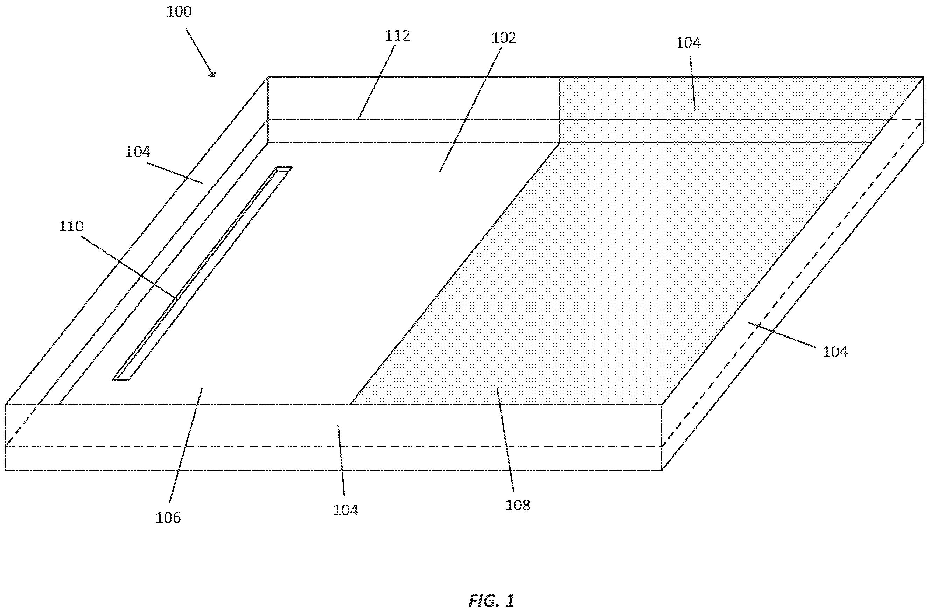

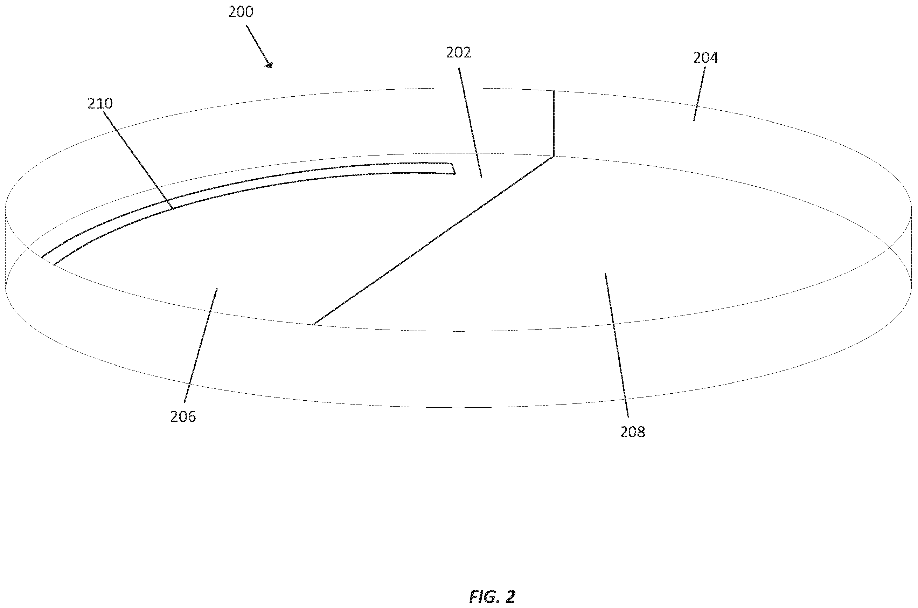

Referring now to Figure 1, Figure 1 shows an example of a fluid-tight container 100 for egg hatching and larvae separation. In this example, the fluid-tight container 100 is made out of acrylic; however, it may be made out of any suitable material. The fluid-tight container 100 includes a base 102 and at least one wall 104 enclosing the base. The base 102 may be square, rectangular, circular (e.g., as shown in Figure 2 and described below), triangular, trapezoidal, or any other shape suitable for the proper functioning of the fluid-tight container 100.

The at least one wall 104 is coupled to the base 102 to form a fluid-tight seal between the base 102 and the wall 104. In this example, the at least one wall 104 is coupled to an edge, or edges depending on the shape, of the base 102; however, the at least one wall 104 may be coupled to any suitable location of the base 102. In some examples, the at least one wall 104 may be coupled to the base 102 by using a glue or a sealant, by welding or melting the base 102 and the at least one wall 104 together, by fastening the base 102 and at least one wall 104 together using various fasteners such as screws, bolts, etc., by molding the base 102 and the at least one wall 104 out of a single piece of material, or by any other suitable method of coupling the at least one wall 104 to the base 102.

A liquid 112 may be included within the fluid-tight container 100. The liquid 112 may have a depth of substantially equal to or less than 0.5 inch or any other suitable depth to permit insect larvae to hatch and move away from any egg hatching debris without drowning in the liquid 112. In some examples, the liquid 112 may be fresh water, saltwater, a solution of water and yeast, or any other suitable liquid 112 or mixture to support the hatching of the larvae.

The base 102 is divided into two contiguous portions, a first portion 106 and a second portion 108 that meet as shown in Figure 1; however, it is not required that the two portions be contiguous. And while the two portions shown in Figure 1 are each approximately half of the base 102, they may make up various proportions of the base 102 other than halves.

The first portion 106 defines a recess 110 into which insect eggs may be deposited and that will generally keep the insect eggs in place. While only a single recess 110 is shown in the example in Figure 1, any suitable number of recesses 110 may be present. The recess 110 may be any suitable shape such as rectangular (e.g., as shown in Figures 1 and 3-8), circular, ovular, trapezoidal, or curved (e.g., as shown in Figure 2) and may have any suitable cross-sectional shape. The recess 110 may also be any suitable depth to keep the insect eggs in place while permitting the hatched larvae to be able to leave the recess 110. Additionally, the recess 110 may extend across an entire dimension of the base 102 (e.g., across the entire width of the base 102) or may extend only partially across a dimension.

In this example, the first portion 106 and the second portion 108 are differently colored: the first portion 106 is white while the second portion 108 is black; however, any suitable color combinations may be employed. As defined by the hue-saturation-value (HSV) color scheme, a color may be said to be white or substantially white if it has a lightness substantially at a maximum HSV lightness value and a minimum saturation value, and a color may be said to be black or substantially black if it has a lightness value substantially at a minimum HSV lightness value. In some examples, the color of the second portion 108 is darker than the color of the first portion 106 meaning that the second color has a color content closer to black than the first color. For example, in the HSV color scheme, one color is darker than another if its brightness value is less than the other color. Other color schemes may be employed in some examples, but some insects are attracted by darkness as it offers a place to hide and repelled by light, thus colors for the portions may be selected to take advantage of such preferences. In this example, the color of the second portion 108 attracts the hatched insect larvae resulting in the hatched larvae moving to the second portion 108 and separating themselves from the egg debris located in the recess 110 and in the first portion 106. It should be understood that the use of the HSV color scheme is merely illustrative, and any suitable coloring scheme to determine relative shadings between different colors may be employed.

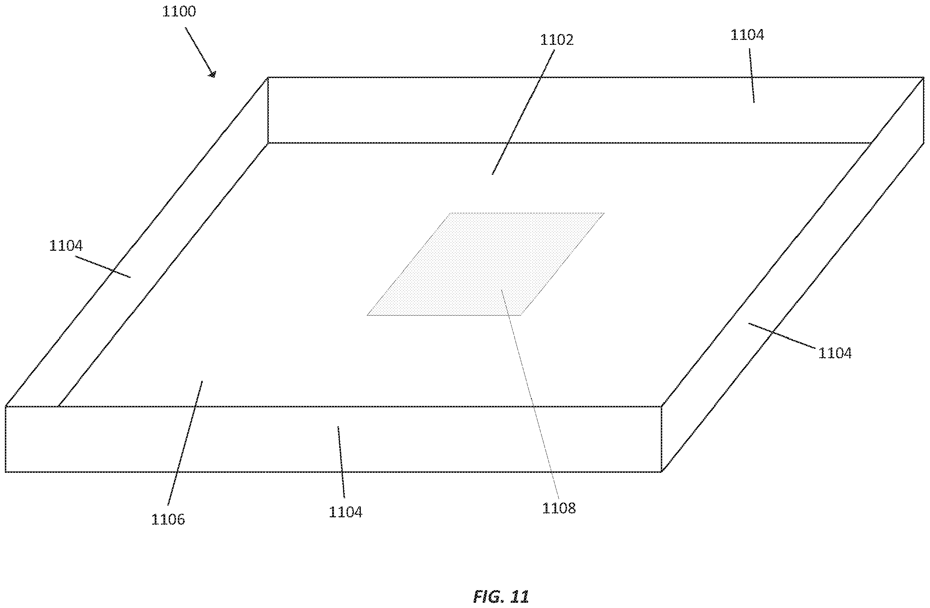

Referring now to Figure 11, Figure 11 shows another example of the fluid-tight container 1100 for egg hatching and larvae separation where the second portion 1108 is surrounded by the first portion 1106. In this example, the first portion 1106 and the second portion 1108 are colored similarly to the first portion 106 and the second portion 108 described above in reference to Figure 1. The insect eggs may be deposited anywhere in the first portion 1106, and the larvae may migrate towards the second portion 1108. Such an arrangement may be advantageous as it may allow a larger region for depositing eggs, with a smaller region from which hatched larvae may be more easily gathered. For example, second portion 1108 may be hinged or in a trapdoor configuration to allow the larvae to be periodically dropped into another container, and then reset to allow additional larvae to migrate onto the second portion.

As discussed above with respect to Figure 1, the fluid-tight container 1100 includes a base 1102, four walls 1104, a first portion 1106, and a second portion 1108 as are described above in relation to Figure 1. In some examples, the first portion 1106 may instead be surrounded by the second portion 1108 in a reverse configuration to what is shown in Figure 11.

Referring now to Figure 2, Figure 2 shows another example of the fluid-tight container 200 for egg hatching and larvae separation. In this example, the fluid-tight container has a circular base 202 with a single wall 204 coupled with and enclosing the base 202 at its edge to form a fluid-tight seal. The fluid-tight container 200 includes two portions, the first portion 206 and the second portion 208, and contains a liquid, as discussed above with respect to Figure 1. Additionally, the first portion 206 defines a curved recess 210. The fluid-tight container 200 may also incorporate a similar color scheme of the first portion 206 and the second portion 208 as discussed above.

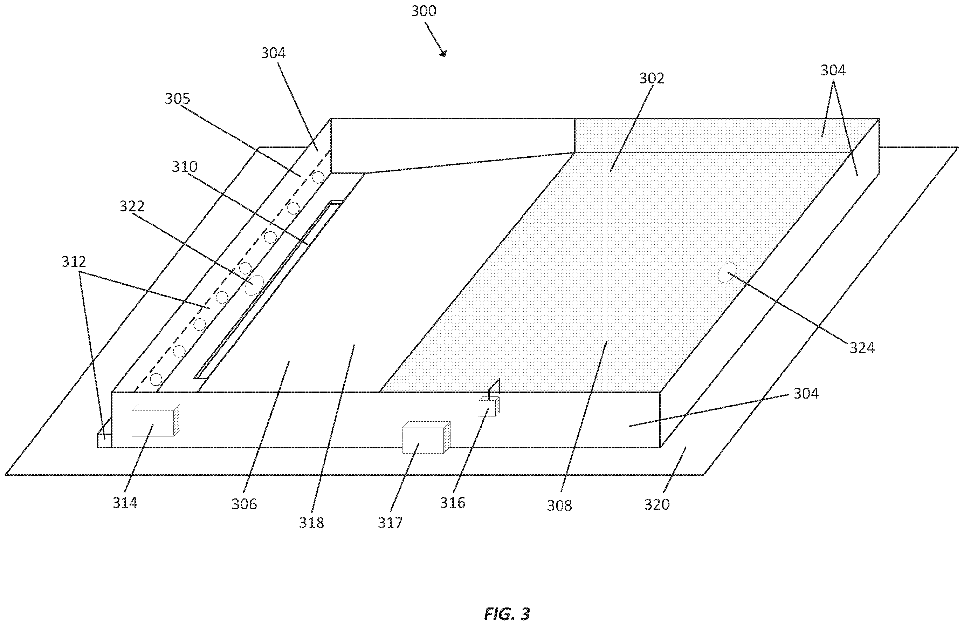

Referring now to Figure 3, Figure 3 shows another example of the fluid-tight container 300 for egg hatching and larvae separation. The fluid-tight container 300 includes a base 302, four walls 304, a first portion 306 colored white, a second portion 308 colored black, and a recess 310 in the first portion 306 as are described above in relation to Figure 1. In this example, the fluid-tight container 300 includes features in addition to those shown in Figure 1 to assist with hatching the larvae and to encourage larvae separation from the egg hatching debris. Some of these features include, for example, a light source 312, a vibration generator 314, a temperature measuring device 316, an inclined slope 318, a heat source 320 and a computing device 317.

In this example, one wall 304 of the fluid-tight container 300 includes a translucent portion 305 located near the first portion 306, though any suitable number of translucent portions 305 may be included in any number of walls 304 in the fluid-tight container 300. As may be seen in Figure 3, the translucent portion 305 extends along an entire wall 304 of the fluid-tight container 300; however, in some examples, the translucent portion 305 may extend only partially along a wall 304. In further examples, the light source 312 may be positioned proximate to the translucent portion(s) 305 of the at least one wall 304 and may be oriented to emit light through the translucent portion 305 into the fluid-tight container 300. The light emitted from the light source 312 into the fluid-tight container 300 may help encourage the insect larvae to move away from the light source 312, and thus move from the first portion 306 to the second portion 308. The light source 312 may be attached to either an inner surface or an outer surface of the fluid-tight container 300 or may be positioned separately from the fluid-tight container 300. In still further examples, the light source 312 may be positioned in any location around the first portion 306 (e.g., above or below the first portion 306 or the recess 310) to emit light into the first portion 306.

In this example, the fluid-tight container 300 includes a vibration generator 314 that outputs a mechanical vibration. The vibration generator 314 may include vibration motors such as eccentric rotating mass (ERM) vibration motors, linear resonant actuators (LRA), an audio speaker, or any other suitable device capable of outputting a mechanical vibration. The vibration generator 314 is positioned to output the mechanical vibration to the first portion 306. For example, in Figure 3 the vibration generator 314 is attached to the outside surface of the first portion 306 of the fluid-tight container 300; however, in some examples, the vibration generator 314 may be attached to the inside surface of the first portion 306 or may be positioned separate from the fluid-tight container 300. The vibration generator 314 is shown attached to one of the at least one walls 304 proximate to the first portion 306, but the vibration generator 314 may also be attached to a plurality of walls 304 or to the base 302. The mechanical vibration generated by the vibration generator 314 and output to the first portion 306 disturbs the insect eggs and larvae located in the recess 310 or the first portion 306 and encourages the larvae to hatch from the eggs and then to separate from the egg hatching debris by moving to the second portion 308.

The fluid-tight container 300 shown in Figure 3 includes a temperature measuring device 316. The temperature measuring device 316 may be any device suitable for measuring the temperature of the liquid found in the fluid-tight container 300, such as a thermometer, thermocouple, infrared sensor, etc. The temperature measuring device 316 is positioned to measure the temperature of the liquid and may display and/or communicate the measured temperature to a user or to the computing device 317, which will be discussed below. In further examples, the temperature measuring device 316 may be attached to the outer surface or inner surface of the fluid-tight container 300, positioned proximate to the fluid-tight container 300, or positioned above the fluid-tight container 300.

In this example, the fluid-tight container 300 includes an inclined slope 318 to assist with the separation of the insect larvae from the egg hatching debris. Some species of insect larvae instinctively climb slopes, thus such a feature may encourage movement of the larvae towards the second portion 308. The inclined slope 318 may be formed as a single unit with the base 302 and/or the at least one wall 304 or the inclined slope 318 may be a separate piece that may be placed into the fluid-tight container 300 or attached to the base 302 and/or the at least one wall 304. In the case where the inclined slope 318 is a separate piece, the inclined slope 318 may be made out of the same material or out of different material than the rest of the fluid-tight container 300. In this example, the inclined slope 318 extends from an edge of the recess 110 to the edge of the second portion 308. In some examples, the inclined slope 318 may begin spaced apart from the recess 110. The base 302 of the second portion 308 is sized such that there is no height difference between the edge of the inclined slope 318 and the second portion 308, though a suitable height difference between the edge of the inclined slope 318 and the second portion 308 may be incorporated into the fluid-tight container. In further examples, the end of the inclined slope 318 may terminate before meeting the second portion 308 such that there is a flat surface of the first portion 306 adjacent to the inclined slope 318 and located between the inclined slope 318 and the second portion 308. The incline of the inclined slope 318 may be any angle suitable for permitting insect larvae to travel across the slope toward the second portion 308.

In this example, the fluid-tight container 300 includes a heat source 320, such as a resistive heating element, a heating pad, an incandescent light bulb, or any other suitable device that may heat the liquid found in the fluid-tight container 300. The heat source 320 may be positioned proximate to or attached to the fluid-tight container 300. For example, the heat source 320 shown in Figure 3 is a heating pad located under the base 302 of the fluid-tight container 300. The heating pad extends under the entire base 302, though in some examples the heating pad may extend only partially under the base 302. The heat source 320 may be positioned to heat the liquid by applying heat to the fluid-tight container 300, as is the case with the heating pad extending under the base 302, or by applying heat directly to the liquid.

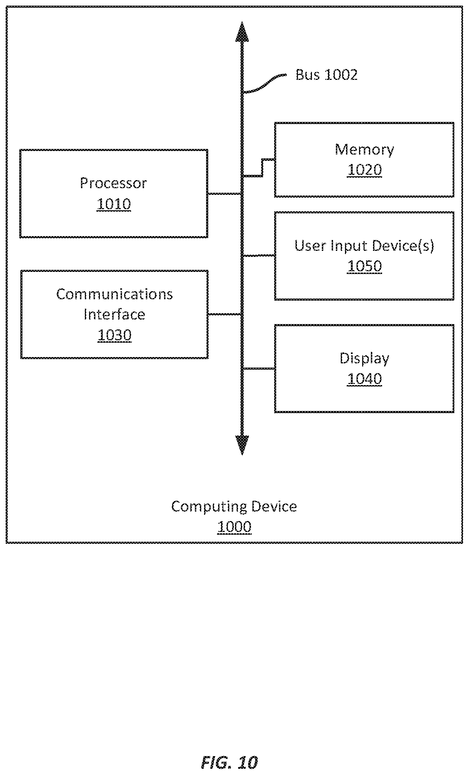

In some examples, the fluid-tight container 300 may include or be controlled by a computing device 317 that may control various features of the fluid-tight container 300. For example, the computing device 317 may turn the light source 312 on or off or adjust the brightness of the light source 312. It may turn the vibration generator 314 on or off or adjust the intensity of the vibration generator 314. The computing device 317 may also turn the heat source 320 on or off or adjust the output of the heat source 320 based on signals and information received by a processor in the computing device 317 from the temperature measuring device 316 in order to regulate the temperature of the liquid found in the fluid-tight container 300. In some examples, however, the computing device 317 may provide more particularized functionality and greater control over devices used with the fluid-tight container 300 such as permitting synchronized application of the various stimuli on a programmed schedule. While Figure 3 only depicts a single computing device 317, it should be appreciated that multiple computing devices 317 may be employed to apportion various processing tasks. Thus, some examples may split processing amongst multiple computing devices 317 to distribute processing requirements. Further details relating to specifics of the computing device 317 are discussed below in relation to Figure 10.

Additionally, chemicals may be used to encourage larvae separation from the egg hatching debris. For example, a chemical deterrent 322, e.g., ethanol or alcohol, may be added to the liquid near the recess 310 or may be applied or attached to a surface of the first portion 306. Or a chemical attractant 324, e.g., a food product, may be added to the liquid found in the second portion 308 or may be applied or attached to a surface of the second portion 308. In some examples, both a chemical deterrent 322 and a chemical attractant 324 may be used to encourage larvae separation.

Many of the features discussed above provide stimuli to encourage larvae separation from egg hatching debris, but examples according to this disclosure are not limited to those examples listed above. It should be understood that any suitable stimuli may be used to help encourage the larvae to separate from the debris. For example, bubbles generated by placing an air pump under the surface of the liquid, cold temperatures generated by an air conditioner or cooling system, shaking generated by the vibration generator 314 or by a moveable base located under the fluid-tight container, mechanical stirring generated using a rod inserted into the liquid, and electric current generated by a generator may all be used as stimuli to encourage larvae separation.

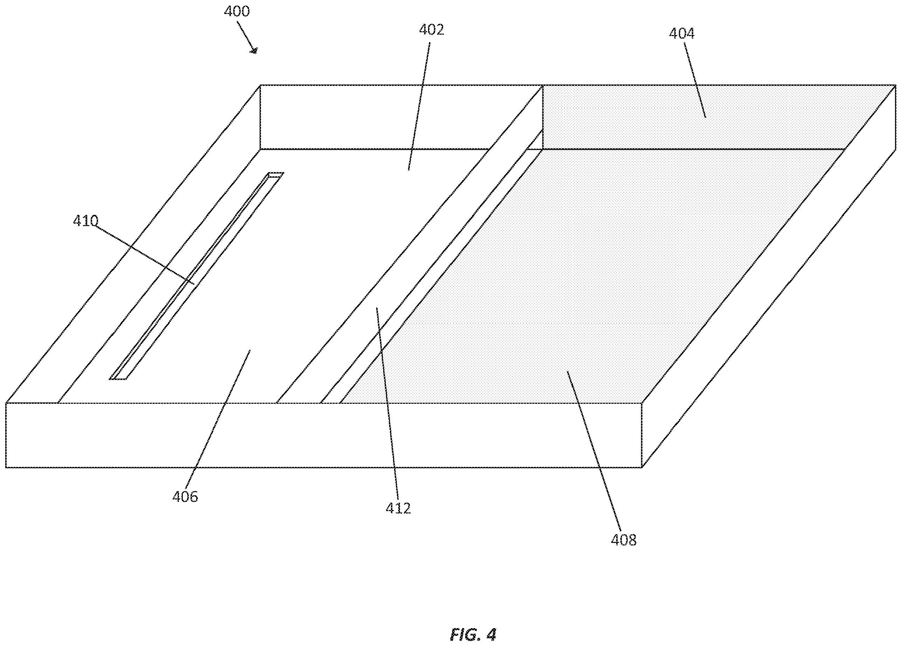

Referring now to Figure 4, Figure 4 shows another example of the fluid-tight container 400 for egg hatching and larvae separation. In this example, the fluid-tight container 400 includes the same features as were discussed in reference to Figure 1. The fluid-tight container 400 includes a base 402, at least one wall 404, a first portion 406, a second portion 408, and a recess 410. Additionally in this example, the fluid-tight container 400 includes a dividing member 412. The dividing member 412 may help with the separation of the insect larvae from the egg hatching debris by permitting insect larvae to move from one side of the dividing member 412 to the other side through a gap between the base 402 and the dividing member 412 while preventing floating insect egg hatching debris from moving past the dividing member 412.

The dividing member 412 extends over the base 402 and between two wall sections of the at least one wall 404. In this example, the dividing member 412 extends over the area of the base 402 were the first portion 406 and the second portion 408 meet. In further examples, the dividing member 412 may extend over only the first portion 406, over only the second portion 408, or over both the first portion 406 and the second portion 408. The dividing member 412 may be formed as a single unit with the two wall sections of the at least one wall 404 or may be attached to the two wall sections after the fluid-tight container 400 is formed. The dividing member 412 is shown in Figure 4 as attached to the two wall sections at right angles. However, it is understood that the dividing member 412 may be attached to the two wall sections at any suitable angle. In some examples, the dividing member 412 may be made out of the same material as the fluid-tight container 400 or out of different material. The dividing member 412 extends from an upper edge of the two wall sections of the at least one wall 404 towards the base 402. However, the dividing member 412 will not contact the base 402. Instead, a gap is formed between a lower end of the dividing member 412 and the base 402. In some examples, the dividing member 412 extends a distance into the liquid.

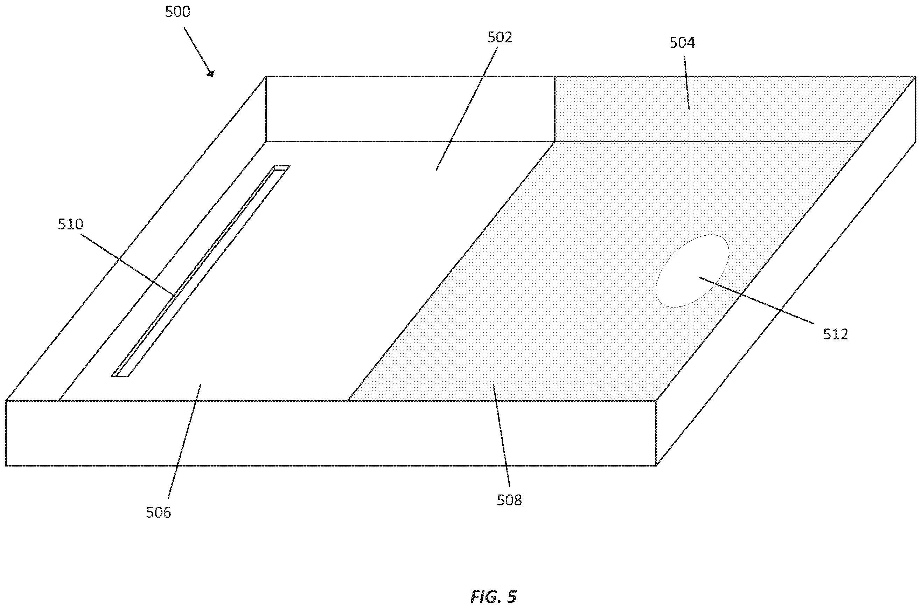

Referring now to Figure 5, Figure 5 shows another example of the fluid-tight container 500 for egg hatching and larvae separation. In this example, the fluid-tight container 500 includes the same features as were discussed above in reference to Figure 1. The fluid-tight container 500 includes a base 502, at least one wall 504, a first portion 506, a second portion 508, and a recess 510. Additionally, the fluid-tight container 500 includes a drain 512, which in this example is a resealable hole. The resealable hole 512 permits the liquid and the insect larvae to be drained from the second portion 508, such as into another container to transport the larvae to a larval rearing container or directly into a larval rearing container. The resealable hole 512 may be suitably sized to permit the liquid and the insect larvae to pass through and may be positioned anywhere in the second portion 508 where the liquid and the insect larvae may still drain through the resealable hole 512. Additionally, the fluid-tight container 500 may include more than one resealable hole 512 to drain the liquid and the insect larvae from the second portion 508. There may also be a resealable hole 512 positioned in the first portion 506 that may help to keep any debris from being drained with the larvae. In some examples, only enough liquid is drained so that the insect larvae are drained from the second portion 508.

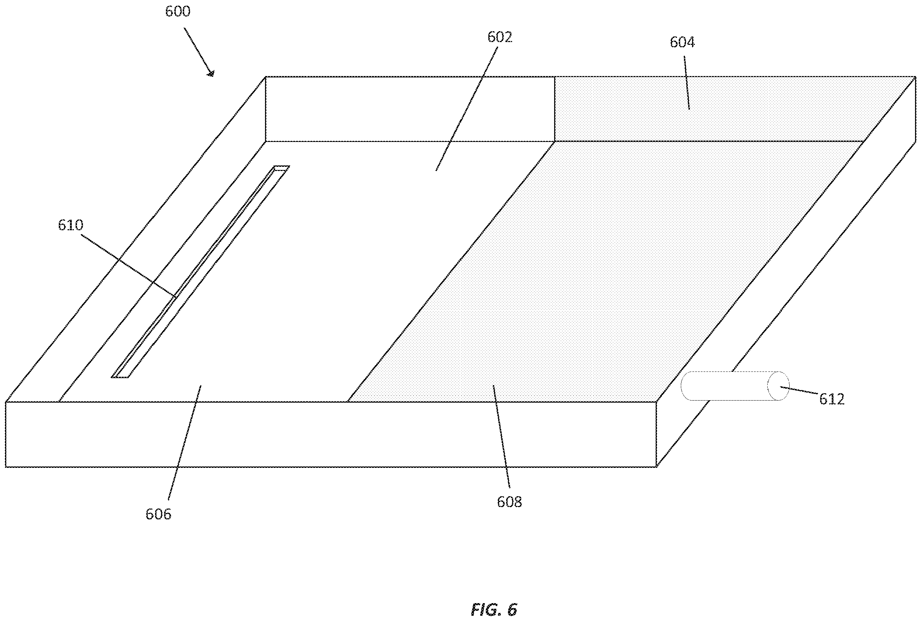

Referring now to Figure 6, Figure 6 shows another example of the fluid-tight container 600 for egg hatching and larvae separation. In this example, the fluid-tight container 600 includes the same features as were discussed above in reference to Figure 1. The fluid-tight container 600 includes a base 602, at least one wall 604, a first portion 606, a second portion 608, and a recess 610. Additionally in this example, the fluid-tight container 600 includes a pump 612. The pump 612 extracts the liquid and the insect larvae from the second portion 608 to another location, such as into another container to transport the larvae to a larval rearing container or directly into a larval rearing container. The pump 612 may be suitably sized to permit the liquid and the insect larvae to pass through and may be positioned anywhere in the second portion 608 where the liquid and the insect larvae may still be extracted through the pump 612. Additionally, the fluid-tight container 600 may include more than one pump 612. Multiple pumps 612 may be positioned in the second portion 608, or pumps 612 may be positioned in both the first portion 606 and the second portion 608 to help keep any debris from being extracted with the larvae. In some examples, only enough liquid is extracted so that the insect larvae are extracted from the second portion 608.

In some examples, the resealable hole 512 discussed in relation to Figure 5 and/or the pump 612 discussed in relation to Figure 6 may be connected to a pipe or a tube that flows past a larvae counter. This may result in an even more efficient process as the larvae would not have to be manually counted

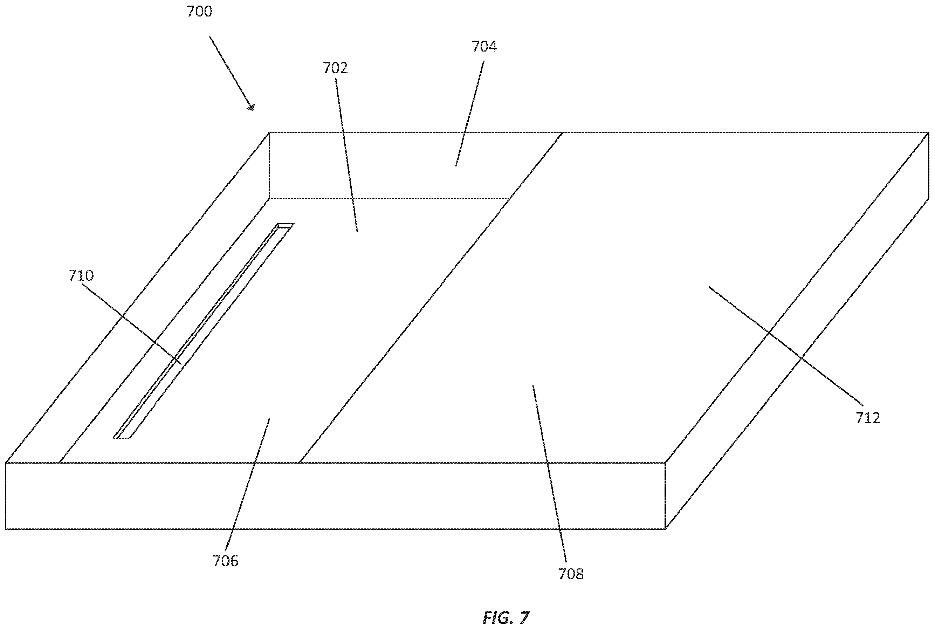

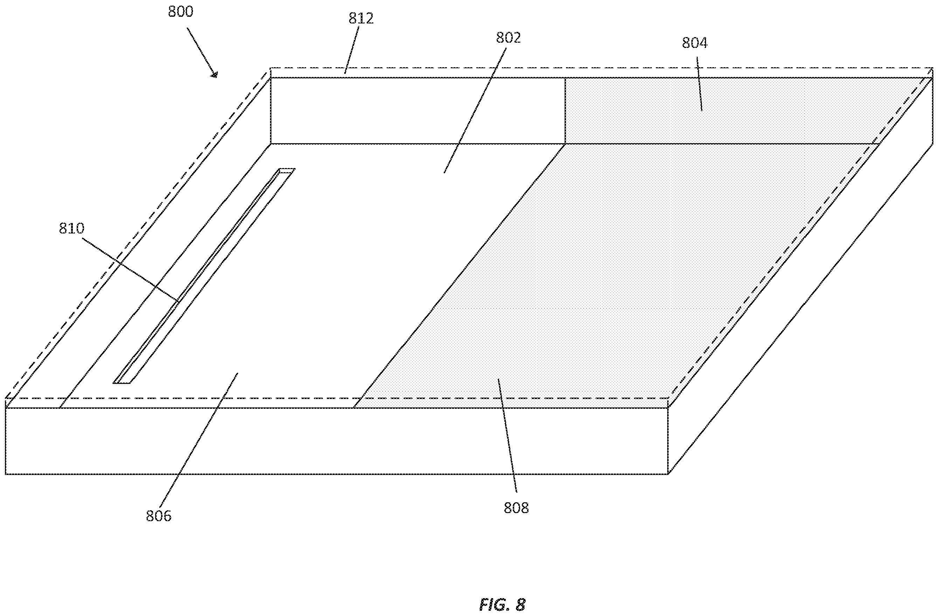

Referring now to Figures 7 and 8, Figures 7 and 8 show additional examples of the fluid-tight container 100 for egg hatching and larvae separation. In the example shown in Figure 7, the fluid-tight container 700 includes the same features as were discussed above in reference to Figure 1. The fluid-tight container 700 includes a base 702, at least one wall 704, a first portion 706, a second portion 708, and a recess 710. Additionally, the fluid-tight container 700 includes an opaque cover 712 to create a darker environment in the second portion 708 and encourage insect larvae to move to the second portion 708 after hatching in the first portion 706. As discussed above, some insect larvae may prefer dark areas to light areas. Thus, the opaque cover 712 may be employed to create a desirable environment for the hatching larvae. The opaque cover 712 may be removable from the fluid-tight container 700 or it may be formed as a single unit with the fluid-tight container 700. Additionally, the opaque cover may extend over the entirety of the second portion 708 or may only partially cover the second portion 708.

In the example shown in Figure 8, the fluid-tight container 800 includes the same features as were discussed above in reference to Figure 1. The fluid-tight container 800 includes a base 802, at least one wall 804, a first portion 806, a second portion 808, and a recess 810. Additionally, the fluid-tight container 800 includes a substantially translucent or transparent cover 812 extending over the entire fluid-tight container 800. The cover 812 may prevent dust, debris, or other contaminants from reaching the liquid and/or the insect larvae. The cover 812 may also serve as an additional surface on which features (e.g., those described in Figure 3) may be attached to the fluid-tight container 800. The cover 812 may be removable from the fluid-tight container 800. The cover 812 may also include openings to permit oxygen flow to the fluid-tight container 800 and/or to permit access to the liquid or interior of the fluid-tight container 800 for any of the features described above in Figure 3, such as the light source 312, the vibration generator 314, the temperature measuring device 316, or the heat source 320, or any other feature that may be included with the fluid-tight container 800.

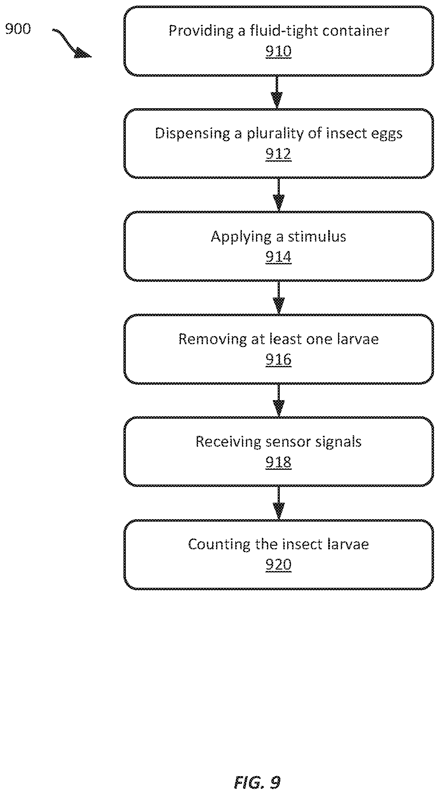

Referring now to Figure 9, Figure 9 shows an example method 900 for separating insect larvae from insect egg hatching debris according to this disclosure. The example method 900 will be discussed with respect to the fluid-tight container 300 shown in Figure 3. However, it should be appreciated that any suitable system for separating insect larvae from insect egg hatching debris may be employed, such as that shown in Figures 1-2, 4-8, or 11.

At block 910, a fluid-tight container 300 and a liquid contained in the fluid-tight container 300, as discussed above in relation to Figure 3, are provided. Here, the fluid-tight container 300 includes a base 302 that has a first portion 306 colored with a first color and defining a recess 310, as discussed above. The base 302 has a second portion 308 that is colored with a second color, which is darker than the first color. The fluid-tight container 300 also includes at least one wall 304 enclosing the base 302 and coupled to the base 302 to form a fluid-tight seal. The first color of the first portion 306 may continue on the at least one wall 304 that is in contact with the first portion 306, and the second color of the second portion 308 may continue on the at least one wall that is in contact with the second portion 308.

At block 912, a plurality of insect eggs are dispensed into the fluid-tight container 300. In some examples, the eggs are dispensed into the recess 310. As discussed above, any suitably sized recess 310 may be incorporated into the first portion 306 to receive the dispensed eggs and permit larvae to hatch from those eggs. The eggs may be dispensed manually by a user or by using an automated machine.

At block 914, additional features may be included with the fluid-tight container 300 to apply a stimulus, or in some examples to apply multiple stimuli, to the fluid-tight container 300 in order to assist with separating larvae from egg hatching debris. For example, light may be emitted by at least one light source 312 positioned to emit light through the translucent portion 305 of at least one wall 304 of the fluid-tight container 300, as discussed above in relation to Figure 3. A mechanical vibration may be output to the first portion 306 using a vibration generator 314, as discussed above. In some examples, a chemical attractant 324 may be introduced to the second portion 308 and/or a chemical deterrent 322 may be introduced to the first portion 306, as discussed above. Additionally, heat may be applied to the fluid-tight container 300 and/or directly to the liquid using a heat source 320, as discussed above in relation to Figure 3.

The computing device 317 may be used to apply the stimulus or adjust the application of the stimulus. For example, the temperature measuring device 316 may output signals that include temperature information that is received by a processor in the computing device 317. The processor may then adjust the heat setting of the heat source 320 based on that temperature information. In some examples, the fluid-tight container 300 may include sensors that are able to monitor the various stimuli applied to the fluid-tight container and send information relating to the stimuli to the processor to permit the computing device 317 to adjust the application of the stimuli.

Due to the construction of the fluid-tight container 300 and the application of any stimuli, the insect larvae are encouraged to separate themselves.

At block 916, at least one larvae hatched from an egg of the plurality of insect eggs dispensed into the fluid-tight container 300 is removed from the second portion 308 of the fluid-tight container 300. As discussed above, after the larvae hatch from the insect eggs dispensed in the first portion 306, they will move to the second portion 308. This is due in part to the coloring and construction of the fluid-tight container 300 as some insect larvae prefer to be in dark areas. Additionally, applying a stimuli, such as light from the light source 312 or mechanical vibration from the vibration generator 314, to the first portion 306 will encourage the larvae to move to the second portion 308 because insect larvae tend to flee from stimulation. The at least one larvae may be removed from the second portion 308 manually by a user, by the resealable drain 512 or the pump 612 as discussed above with respect to Figures 5 and 6 respectively, or by any other suitable method of removing larvae from the fluid-tight container 300.

For example, referring again to Figure 5, the larvae may be removed from the second portion 508 by draining, via the resealable drain 512, the larvae and some of the liquid from the fluid-tight container 500. As a further example, referring to Figure 6, the larvae may be removed from the second portion 608 by pumping out, via the pump 612, the larvae and some of the liquid from the fluid-tight container 600.

At block 918, a processor 1010, discussed in further detail below in relation to Figure 10, receives at least one sensor signal from a sensor indicating larvae are being removed from the second portion 308. For example, a flow cytometer may be used to detect the larvae as they flow past a laser. Additionally, a light curtain may be used to detect the larvae as the as they flow through and break up the light beams.

At block 920, the processor 1010 counts the larvae based on the sensor signals. In some examples, the processor 1010 increments a counter based on each sensed larvae passing the sensor. In some examples, the processor 1010 resets its counter before a new batch of larvae are hatched in the fluid-tight container 300; however, in some examples, the processor 1010 may maintain a running count of all sensed larvae from multiple batches or larvae.

Referring now to Figure 10, Figure 10 shows an example computing device 1000 suitable for use in example devices or methods for egg hatching and larvae separation according to this disclosure. The example computing device 1000 includes a processor 1010 which is in communication with the memory 1020 and other components of the computing device 1000 using one or more communications buses 1002. The processor 1010 executes processor-executable instructions stored in the memory 1020 to assist with egg hatching and larvae separation, such as instructions for part or all of the example method 900 described above with respect to Figure 9. The computing device 1000, in this example, also includes one or more user input devices 1050, such as a keyboard, mouse, touchscreen, microphone, etc., to accept user input. The computing device 1000 also includes a display 1040 to provide visual output to a user.

The computing device 1000 also includes a communications interface 1030. In some examples, the communications interface 1030 may enable communications using one or more networks, including a local area network ("LAN"); wide area network ("WAN"), such as the Internet; metropolitan area network ("MAN"); point-to-point or peer-to-peer connection; etc. Communication with other devices may be accomplished using any suitable networking protocol. For example, one suitable networking protocol may include the Internet Protocol ("IP"), Transmission Control Protocol ("TCP"), User Datagram Protocol ("UDP"), or combinations thereof, such as TCP/IP or UDP/IP.

While some examples of methods and devices herein are described in terms of software executing on various machines, the methods and devices may also be implemented as specifically-configured hardware, such as field-programmable gate array (FPGA) specifically to execute the various methods. For example, examples can be implemented in digital electronic circuitry, or in computer hardware, firmware, software, or in a combination thereof. In one example, a device may include a processor or processors. The processor includes a computer-readable medium, such as a random access memory (RAM) coupled to the processor. The processor executes computer-executable program instructions stored in memory, such as executing one or more computer programs. Such processors may include a microprocessor, a digital signal processor (DSP), an application-specific integrated circuit (ASIC), field programmable gate arrays (FPGAs), and state machines. Such processors may further include programmable electronic devices such as PLCs, programmable interrupt controllers (PICs), programmable logic devices (PLDs), programmable read-only memories (PROMs), electronically programmable read-only memories (EPROMs or EEPROMs), or other similar devices.

Such processors may include, or may be in communication with, media, for example computer-readable storage media, that may store instructions that, when executed by the processor, can cause the processor to perform the steps described herein as carried out, or assisted, by a processor. Examples of computer-readable media may include, but are not limited to, an electronic, optical, magnetic, or other storage device capable of providing a processor, such as the processor in a web server, with computer-readable instructions. Other examples of media include, but are not limited to, a floppy disk, CD-ROM, magnetic disk, memory chip, ROM, RAM, ASIC, configured processor, all optical media, all magnetic tape or other magnetic media, or any other medium from which a computer processor can read. The processor, and the processing, described may be in one or more structures, and may be dispersed through one or more structures. The processor may include code for carrying out one or more of the methods (or parts of methods) described herein.

The foregoing description of some examples has been presented only for the purpose of illustration and description and is not intended to be exhaustive or to limit the disclosure to the precise forms disclosed. Numerous modifications and adaptations thereof will be apparent to those skilled in the art without departing from the scope of the disclosure.

Reference herein to an example or implementation means that a particular feature, structure, operation, or other characteristic described in connection with the example may be included in at least one implementation of the disclosure. The disclosure is not restricted to the particular examples or implementations described as such. The appearance of the phrases "in one example," "in an example," "in one implementation," or "in an implementation," or variations of the same in various places in the specification does not necessarily refer to the same example or implementation. Any particular feature, structure, operation, or other characteristic described in this specification in relation to one example or implementation may be combined with other features, structures, operations, or other characteristics described in respect of any other example or implementation.

Use herein of the word "or" is intended to cover inclusive and exclusive OR conditions. In other words, A or B or C includes any or all of the following alternative combinations as appropriate for a particular usage: A alone; B alone; C alone; A and B only; A and C only; B and C only; and A and B and C.

## Claims

1. A device for egg hatching and larvae separation comprising a fluid-tight container (100, 300), wherein the fluid-tight container comprises:
a base (102, 302) having a first portion (106, 306) and a second portion (108, 308);
at least one wall (104, 304) enclosing the base and coupled to the base to form a fluid-tight seal;
wherein the first portion of the base comprises a first color and defines a recess (110, 310);
wherein the recess (110,310) is arranged for depositing of and keeping in place insect eggs for hatching in the first recess (110, 310);
wherein the second portion of the base comprises a second color darker than the first color;
wherein the color of the second portion (108, 308) is adapted to attract hatched insect larvae so that the hatched larvae move to the second portion (108, 308) and separate themselves from the egg debris located in the recess (110, 310) and in the first portion (106, 306).

2. The device of claim 1, wherein a liquid (112) is disposed within the fluid-tight container.

3. The device of claim 2, further comprising a heat source (320) positioned to maintain a predetermined temperature of the liquid disposed in the fluid-tight container.

4. The device of claim 3, further comprising a temperature measuring device (316) configured to measure and regulate the temperature of the liquid disposed in the fluid-tight container.

5. The device of any preceding claim, wherein:
the base comprises a rectangular shape; and/or
the base and at least one wall comprise a single molded piece of material; and/or
the first color comprises white and the second color comprises black; and/or
the first portion and the second portion are contiguous.

6. The device of any preceding claim, wherein:
a portion (305) of the at least one wall is translucent and a light source (312) is positioned and oriented to emit light through the translucent portion into the fluid-tight container; and/or
the first portion defines a slope (318) inclining from the recess to the second portion; and/or
the device further comprises a vibration generator (314) to output a mechanical vibration to the first portion; and/or
the device further comprises a chemical deterrent (322) located in the first portion and/or a chemical attractant (324) located in the second portion.

7. The device of claim 1, wherein the at least one wall includes two wall sections, wherein a dividing member (412):
extends over the base between the two wall sections, and
defines a gap between the base and the dividing member,
wherein the dividing member is configured to permit insect larvae to pass from one side of the member to the other side of the member and prevent an insect egg hatching debris from passing from one side of the member to the other side of the member.

8. The device of claim 1, wherein the second portion defines a resealable hole (512) to drain a liquid and a plurality of insect larvae from the second portion.

9. The device of claim 1, wherein the fluid-tight container further comprises a pump (612) configured to extract a liquid and a plurality of insect larvae from the second portion.

10. The device of claim 1, wherein the fluid-tight container further comprises a removable opaque cover (712) or a removable substantially translucent or transparent cover (812).

11. The device of claim 4, wherein at least a portion (305) of the at least one wall is translucent; and
the device further comprises:
a light source (312) positioned and oriented to transmit light through the translucent portion of the at least one wall into the fluid-tight container;
a vibration generator (314) coupled to the fluid-tight container to output a mechanical vibration to the first portion; and
a processor (317) configured to execute processor executable instructions stored in a memory to:
activate and deactivate the light source,
activate and deactivate the vibration generator,
receive a signal from the temperature measuring device, and
output a signal to the heat source based on the signal from the temperature measuring device.

12. A method for egg hatching and larvae separation comprising,
providing a device comprising a fluid-tight container according to any preceding claim, in which a liquid is disposed in the fluid-tight container;
dispensing a plurality of insect eggs into the recess of the fluid-tight container; and
removing at least one larvae from the second portion of the fluid-tight container, the at least one larvae having hatched from an insect egg of the plurality of insect eggs.

13. The method of claim 12, further comprising:
receiving, by a processor from a sensor, sensor signals indicating insect larvae being removed from the second portion; and
counting, by the processor, the insect larvae based on the sensor signals.

14. The method of claim 12, further comprising any one or more of:
emitting light, by at least one light source positioned and oriented to emit light, through a translucent portion of the at least one wall into the fluid-tight container;
outputting a mechanical vibration to the first portion using a vibration generator' introducing a chemical attractant to the second portion;
introducing a chemical deterrent to the first portion;
applying heat from a heat source to the fluid-tight container; and
monitoring the temperature of the liquid using a temperature measuring device.

15. The method of claim 12, wherein removing the at least one larvae from the second portion comprises:
draining the at least one larvae from the fluid-tight container; or
pumping out the liquid and the at least one larvae from the fluid-tight container.

## Patentansprüche

1. Vorrichtung zur Trennung von Bruteiern und Larven, umfassend einen fluiddichten Behälter (100, 300), wobei der fluiddichte Behälter Folgendes umfasst:
eine Basis (102, 302) mit einem ersten Abschnitt (106, 306) und einem zweiten Abschnitt (108, 308);
zumindest eine Wand (104, 304), die die Basis umschließt und mit der Basis gekoppelt ist, um eine fluiddichte Dichtung zu bilden;
wobei der erste Abschnitt der Basis eine erste Farbe umfasst und eine Vertiefung (110, 310) definiert;
wobei die Vertiefung (110, 310) zum Ablegen und Aufbewahren von Insekteneiern zum Bebrüten in der ersten Vertiefung (110, 310) angeordnet ist;
wobei der zweite Abschnitt der Basis eine zweite Farbe umfasst, die dunkler ist als die erste Farbe;
wobei die Farbe des zweiten Abschnitts (108, 308) ausgelegt ist, um geschlüpfte Insektenlarven anzuziehen, sodass sich die geschlüpften Larven in den zweiten Abschnitt (108, 308) bewegen und sich von den Eierresten, die sich in der Vertiefung (110, 310) und in dem ersten Abschnitt (106, 306) befinden, trennen.

2. Vorrichtung nach Anspruch 1, wobei eine Flüssigkeit (112) innerhalb des fluiddichten Behälters angeordnet ist.

3. Vorrichtung nach Anspruch 2, ferner umfassend eine Wärmequelle (320), die angeordnet ist, um eine vorbestimmte Temperatur der Flüssigkeit, die in dem fluiddichten Behälter angeordnet ist, aufrechtzuerhalten.

4. Vorrichtung nach Anspruch 3, ferner umfassend eine Temperaturmessvorrichtung (316), die ausgelegt ist, um die Temperatur der Flüssigkeit, die in dem fluiddichten Behälter angeordnet ist, zu messen und zu regeln.

5. Vorrichtung nach einem der vorangegangenen Ansprüche, wobei:
die Basis eine rechteckige Form umfasst; und/oder
die Basis und zumindest eine Wand ein einzelnes Formteil aus einem Material umfassen; und/oder
die erste Farbe weiß umfasst und die zweite Farbe schwarz umfasst; und/oder
der erste Abschnitt und der zweite Abschnitt durchgängig sind.

6. Vorrichtung nach einem der vorangegangenen Ansprüche, wobei:
ein Abschnitt (305) der zumindest einen Wand transluzent ist und eine Lichtquelle (312) angeordnet und ausgerichtet ist, um Licht durch den transluzenten Abschnitt in den fluiddichten Behälter auszustrahlen; und/oder
der erste Abschnitt eine Abschrägung (318) definiert, die sich von der Vertiefung bis zum zweiten Abschnitt neigt; und/oder
die Vorrichtung ferner einen Schwingungsgenerator (314) umfasst, um eine mechanische Schwingung an den ersten Abschnitt auszugeben; und/oder
die Vorrichtung ferner ein chemisches Abschreckungsmittel (322) umfasst, das sich in dem ersten Abschnitt befindet, und/oder ein chemisches Anziehungsmittel (324) umfasst, das sich in dem zweiten Abschnitt befindet.

7. Vorrichtung nach Anspruch 1, wobei die zumindest eine Wand zwei Wandabschnitte umfasst, wobei ein Trennelement (412):
sich über die Basis zwischen den zwei Wandabschnitten erstreckt und
einen Spalt zwischen der Basis und dem Trennelement definiert,
wobei das Trennelement ausgelegt ist, um zuzulassen, dass Insektenlarven von einer Seite des Elements auf die andere Seite des Elements gelangen können, und um zu verhindern, dass Insektenei-Schlüpfreste von der einen Seite des Elements auf die andere Seite des Elements gelangen.

8. Vorrichtung nach Anspruch 1, wobei der zweite Abschnitt ein wiederverschließbares Loch (512) definiert, um eine Flüssigkeit und eine Vielzahl von Insektenlarven aus dem zweiten Abschnitt abzuführen.

9. Vorrichtung nach Anspruch 1, wobei der fluiddichte Behälter ferner eine Pumpe (612) umfasst, die ausgelegt ist, um eine Flüssigkeit und eine Vielzahl von Insektenlarven aus dem zweiten Abschnitt zu extrahieren.

10. Vorrichtung nach Anspruch 1, wobei der fluiddichte Behälter ferner eine abnehmbare undurchsichtige Abdeckung (712) oder eine im Wesentlichen transluzente oder durchsichtige Abdeckung (812) umfasst.

11. Vorrichtung nach Anspruch 4, wobei zumindest ein Teil (305) der zumindest einen Wand transluzent ist; und
die Vorrichtung ferner Folgendes umfasst:
eine Lichtquelle (312), die angeordnet und ausgerichtet ist, um Licht durch den transluzenten Abschnitt in den fluiddichten Behälter auszustrahlen;
einen Schwingungsgenerator (314), der mit dem fluiddichten Behälter gekoppelt ist, um eine mechanische Schwingung an den ersten Abschnitt auszugeben; und
einen Prozessor (317), der ausgelegt ist, um prozessorausführbare Befehle, die in einem Speicher gespeichert sind, auszuführen, um:
die Lichtquelle zu aktivieren und zu deaktivieren,
den Schwingungsgenerator zu aktivieren und zu deaktivieren,
ein Signal von der Temperaturmessvorrichtung zu empfangen und
ein Signal basierend auf dem Signal von der Temperaturmessvorrichtung an die Wärmequelle auszugeben.

12. Verfahren zur Trennung von Bruteiern und Larven, umfassend:
das Bereitstellen einer Vorrichtung, umfassend einen fluiddichten Behälter nach einem der vorangegangenen Ansprüche, in der eine Flüssigkeit in dem fluiddichten Behälter angeordnet ist;
das Ablegen einer Vielzahl von Insekteneiern in die Vertiefung des fluiddichten Behälters; und
das Entfernen zumindest einer Larve aus dem zweiten Abschnitt des fluiddichten Behälters, wobei die zumindest eine Larve aus einem Insektenei aus der Vielzahl von Insekteneiern geschlüpft ist.

13. Verfahren nach Anspruch 12, das ferner Folgendes umfasst:
Empfangen von Sensorsignalen von einem Sensor durch einen Prozessor, die angeben, dass Insektenlarven aus dem zweiten Abschnitt entnommen werden; und
Zählen der Insektenlarven basierend auf den Sensorsignalen durch den Prozessor.

14. Verfahren nach Anspruch 12, das ferner ein beliebiges oder mehrere der Folgenden umfasst:
Ausstrahlen von Licht durch einen transluzenten Abschnitt der zumindest einen Wand durch die zumindest eine Lichtquelle, die angeordnet und ausgerichtet ist, um Licht auszustrahlen, in den fluiddichten Behälter;
Ausgeben einer mechanischen Schwingung an den ersten Abschnitt unter Verwendung eines Schwingungsgenerators;
Einführen eines chemischen Anziehungsmittels in den zweiten Abschnitt;
Einführen eines chemischen Abschreckungsmittels in den ersten Abschnitt;
Anwenden von Wärme von einer Wärmequelle auf den fluiddichten Behälter; und
Überwachen der Temperatur der Flüssigkeit unter Verwendung einer Temperaturmessvorrichtung.

15. Verfahren nach Anspruch 12, wobei das Entfernen der zumindest einen Larve aus dem zweiten Abschnitt Folgendes umfasst:
Abführen der zumindest einen Larve aus dem fluiddichten Behälter; oder
Abpumpen der Flüssigkeit und der zumindest einen Larve aus dem fluiddichten Behälter.

## Revendications

1. Dispositif pour l'éclosion des oeufs et la séparation de larves, comprenant un récipient étanche aux fluides (100, 300), dans lequel le récipient étanche aux fluides comprend :
une base (102, 302) présentant une première partie (106, 306) et une seconde partie (108, 308) ;
au moins une paroi (104, 304) entourant la base et couplée à la base pour former un joint étanche aux fluides ;
dans lequel la première partie de la base comprend une première couleur et définit un évidement (110, 310) ;
dans lequel l'évidement (110, 310) est agencé pour déposer et maintenir en place des oeufs d'insecte pour l'éclosion dans le premier évidement (110, 310) ;
dans lequel la seconde partie de la base comprend une seconde couleur plus sombre que la première couleur ;
dans lequel la couleur de la seconde partie (108, 308) est adaptée pour attirer les larves d'insecte écloses de sorte que les larves écloses se déplacent vers la seconde partie (108, 308) et se séparent des débris d'oeufs situés dans l'évidement (110, 310) et dans la première partie (106, 306) .

2. Dispositif selon la revendication 1, dans lequel un liquide (112) est disposé à l'intérieur du récipient étanche aux fluides.

3. Dispositif selon la revendication 2, comprenant en outre une source de chaleur (320) positionnée pour maintenir une température prédéterminée du liquide disposé dans le récipient étanche aux fluides.

4. Dispositif selon la revendication 3, comprenant en outre un dispositif de mesure de température (316) configuré pour mesurer et réguler la température du liquide disposé dans le récipient étanche aux fluides.

5. Dispositif selon l'une quelconque des revendications précédentes, dans lequel :
la base comprend une forme rectangulaire ; et/ou
la base et au moins une paroi comprennent une pièce de matériau moulée unique ; et/ou
la première couleur comprend du blanc et la seconde couleur comprend du noir ; et/ou
la première partie et la seconde partie sont contiguës.

6. Dispositif selon l'une quelconque des revendications précédentes, dans lequel :
une partie (305) de la au moins une paroi est translucide et une source de lumière (312) est positionnée et orientée pour émettre de la lumière à travers la partie translucide jusque dans le récipient étanche aux fluides ; et/ou
la première partie définit une pente (318) s'inclinant de l'évidement à la seconde partie ; et/ou
le dispositif comprend en outre un générateur de vibration (314) pour délivrer en sortie une vibration mécanique vers la première partie ; et/ou
le dispositif comprend en outre un agent de dissuasion chimique (322) situé dans la première partie et/ou un répulsif chimique (324) situé dans la seconde partie.

7. Dispositif selon la revendication 1, dans lequel la au moins une paroi inclut deux sections de paroi, dans lequel un élément de division (412) :
s'étend au-dessus de la base entre les deux sections de paroi, et
définit un espace entre la base et l'élément de division,
dans lequel l'élément de séparation est configuré pour permettre à des larves d'insecte de passer d'un premier côté de l'élément à l'autre côté de l'élément et empêcher des débris d'éclosion d'oeufs d'insecte de passer du premier côté de l'élément à l'autre côté de l'élément.

8. Dispositif selon la revendication 1, dans lequel la seconde partie définit un trou pouvant être refermé de manière étanche (512) pour drainer un liquide et une pluralité de larves d'insecte à partir de la seconde partie.

9. Dispositif selon la revendication 1, dans lequel le récipient étanche aux fluides comprend en outre une pompe (612) configurée pour extraire un liquide et une pluralité de larves d'insecte à partir de la seconde partie.

10. Dispositif selon la revendication 1, dans lequel le récipient étanche aux fluides comprend en outre un couvercle opaque amovible (712) ou un couvercle sensiblement translucide ou transparent amovible (812).

11. Dispositif selon la revendication 4, dans lequel au moins une partie (305) de la au moins une paroi est translucide ; et
le dispositif comprenant en outre :
une source de lumière (312) positionnée et orientée pour transmettre de la lumière à travers la partie translucide de la au moins une paroi dans le récipient étanche aux fluides ;
un générateur de vibration (314) couplé au récipient étanche aux fluides pour délivrer une vibration mécanique à la première partie ; et
un processeur (317) configuré pour exécuter des instructions exécutables par le processeur stockées dans une mémoire pour :
activer et désactiver la source de lumière,
activer et désactiver le générateur de vibrations,
recevoir un signal provenant du dispositif de mesure de température, et
émettre un signal vers la source de chaleur sur la base du signal provenant du dispositif de mesure de température.

12. Procédé d'éclosion d'oeufs et de séparation de larves, comprenant les étapes consistant à :
fournir un dispositif comprenant un récipient étanche aux fluides selon l'une quelconque des revendications précédentes, dans lequel un liquide est disposé dans le récipient étanche aux fluides ;
distribuer une pluralité d'oeufs d'insecte dans l'évidement du récipient étanche aux fluides ; et
retirer au moins une larve de la seconde partie du récipient étanche aux fluides, la au moins une larve ayant éclos à partir d'un oeuf d'insecte de la pluralité d'oeufs d'insectes.

13. Procédé selon la revendication 12, comprenant en outre les étapes consistant à :
recevoir, par l'intermédiaire d'un processeur à partir d'un capteur, des signaux de capteur indiquant que des larves d'insecte sont retirées de la seconde partie ; et
compter, par l'intermédiaire du processeur, les larves d'insecte sur la base des signaux de capteur.

14. Procédé selon la revendication 12, comprenant en outre une quelconque ou plusieurs parmi les étapes consistant à :
émettre de la lumière, par l'intermédiaire d'au moins une source de lumière positionnée et orientée pour émettre de la lumière, à travers une partie translucide de la au moins une paroi dans le récipient étanche aux fluides ;
délivrer en sortie une vibration mécanique vers la première partie en utilisant un générateur de vibration,
introduire un attractif chimique dans la seconde partie ;
introduire un répulsif chimique dans la première partie ;
appliquer de la chaleur provenant d'une source de chaleur au récipient étanche aux fluides ; et
surveiller la température du liquide en utilisant un dispositif de mesure de température.

15. Procédé selon la revendication 12, dans lequel l'élimination de la au moins une larve à partir de la seconde partie comprend les étapes consistant à :
vidanger la au moins une larve à partir du récipient étanche aux fluides ; ou
pomper le liquide et la au moins une larve à partir du récipient étanche aux fluides.
